# EUROPEAN PATENT APPLICATION

(11) **EP 2 518 504 A1**
(43) Date of publication of application: **31.10.2012**
(21) Application number: 11164382.1
(22) Date of filing: 29.04.2011
(51) Int. Cl.: G01N 33/543, G01N 33/569

(54) **A method for the quantitative and qualitative characterization of antigen-specific T cells recognizing a specific antigen**

(71) Applicant: Miltenyi Biotec GmbH, 51429 Bergisch Gladbach (DE)
(72) Inventor: Scheffold, Alexander, 51069 Köln (DE); Bacher, Petra, 51379 Leverkusen (DE)
(74) Representative: Kisters, Michael Marcus

(57) **Abstract**

The invention relates to a method for sensitive quantitative and/or qualitative analysis of target T cells comprising the steps a) enrichment of said cells from a mixture of said cells and other cells in a sample by the use of one or more activation markers expressed on antigen-activated T cells in a parallel cell sorting process and b) analysis of the cells of step a).

The invention relates also to a method of diagnosing diseases based on the analysis of the target T cells.

## Description

### Field of the invention

The invention relates to a method for the qualitative and quantitative characterization of antigen-specific T cells recognizing a specific antigen in body fluids by the use of antigen-specific T cell activation markers.

### Background of the invention

T cells are the central organizers and effectors of the immune system and are responsible for effective immunity against pathogens and tumors as well as for keeping unresponsiveness (tolerance) against autoantigens and harmless non-self antigens, such as food. To achieve this goal T cells are educated either during their early development in the thymus or later on in the periphery to acquire distinct effector functions, which can be stably inherited from a single cell to its progeny and in this way contribute to immunological memory. The type of effector function a certain T cell, specific for a defined antigen has acquired, may determine the outcome of the immune response and may therefore have high diagnostic or prognostic value for immune mediated diseases, infections or cancer. The type of T lymphocyte activation and differentiation into certain functional distinct populations is determined by co-stimulatory activation signals from antigen-presenting cells. Activation signals are represented by ligands for receptors of T lymphocytes. Said ligands are situated on the surface of the APCs, they are bound to the extracellular matrix or secreted by cells, as are the cytokines. However, in addition to antigen-specific activation by signals via the antigen receptor of the T lymphocytes and co-stimulating ligands, non-specific activation of T lymphocytes has also been described, e.g. via cytokines or lectins.

Antigen-specific T cells direct the immune response against pathogens expressing said antigen. To cope with the almost unrestricted diversity of antigens, a single individuum might be confronted with, the immune system generates a high diversity of receptors for T and B lymphocytes. Due to this high diversity the frequency of a single specificity is rather low and probably in the range of 1 in 10⁶ (Alanio et al. 2010, ; Moon et al., 2007) in the naïve immune system. Upon encounter with the antigen a single reactive clone can strongly expand. After clearance of the infection the size of the clonally expanded cells contracts and usually the frequency for antigen-specific memory cells ranges from 10⁻⁵ - 1%. Information about the frequency phenotype and functional capabilities of the rare antigen-specific T cells may have important diagnostic or prognostic value for infections, tumors or immune mediated diseases, such as autoimmune diseases. However, the quantitative and qualitative characterization of rare antigen-specific T lymphocytes recognizing a specific antigen is only possible with some imprecision with the present state of the art. Technologies have been described to identify antigen-specific T cells, based on specific antigen binding, e.g. multimeric complexes of peptide-loaded MHC molecules (Altman and Davis, 2003; Sims et al. 2010), or based on expression of "activation" markers, e.g. cytokines, CD137 (Wehler et al., 2008; Wolfl et al., 2008; Wolfl et al., 2007), CD154 (Chattopadhyay et al., 2005; Frentsch et al., 2005; Kirchhoff et al., 2007; Thiel et al., 2004), upon in vitro activation with the specific antigen. Multimers have the limitation that the respective antigen and MHC allele have to be exactly defined, which is rarely the case, especially for complex pathogenic organisms such as bacteria, virus, fungi or multicellular parasites, which may express thousands of potential antigens. Furthermore only few MHC class II multimers for the detection of antigen-specific T helper cells have been described. In contrast, activation markers can be used for CD8 and CD4 T cells and are not restricted to certain MHC alleles, e.g. T cells reactive to a certain antigen independent of exactly defined peptide epitopes and HLA-restriction can be identified by antigen-induced expression of activation markers such as cytokine secretion (Campbell et al., 2011) CD137 or CD154 (Chattopadhyay et al., 2005; Frentsch et al., 2005; Kirchhoff et al., 2007; Thiel et al., 2004; Wehler et al., 2008; Wolfl et al., 2008; Wolfl et al., 2007) WO2004/027428; EP appl. no. 10175578) Any suitable antigen can be used for stimulation, i.e. peptides, proteins, superantigens, pathogen or target cell lysates, or antigen-presenting cells transfected with antigen coding mRNA or antigen expression plasmids.

However, for rare antigen-specific T cells recognizing a specific antigen, comprehensive information about the frequency, phenotype and antigen-specificity of these cells in healthy donors and about disease associated changes are missing.

Due to their low frequency most T cell analyses especially those focussing on CD4 T cell responses, e.g. for the detection of rare autoantigen-specific, tumor-specific or fungi-reactive T cells employed long-lasting in vitro culture and/or population-based methods (³H-thymidin incorporation, ELISA) or ELISPOT (Bozza et al., 2009; Chai et al., ; Chaudhary et al., ; Day et al., 2003; Hebart et al., 2002; Nepom, 2005; Perruccio et al., 2005; Potenza et al., 2007; Vollers and Stem, 2008; Warris et al., 2005; Wolfl et al., 2008; Wolfl et al., 2007)), providing limited information about the correlation of frequency, phenotype and function of the reactive cells. These methods also suffer from prolonged *in vitro* manipulation of the reactive T cells making it difficult to draw conclusions about the phenotype or function of the T cells directly ex vivo, i.e. at the onset of the in vitro culture. If cytokine production is taken as a read-out the analysis is restricted to a certain cytokine producing subsets, which may represent only a fraction of all reactive T cells.

Despite their low frequency the antigen-specific T cells are regarded as key players orchestrating or executing immune responses and to be relevant for the ethiopathogenesis of multiple autoimmune diseases, tumors, infectious diseases. Thus understanding the frequency, phenotype and function of these rare events, i.e. obtaining statistically significant data, is a major problem for diagnostic or prognostic evaluation of T cell immunity in these clinical situations (Janeway's Immunobiology, 7th edition 2008, Chapter 13, 14.8-14.17, 15.14-15. 18). Despite their central role the direct ex vivo analysis of autoreactive or tumor reactive T cells is so far not routinely be used, due to the technical limitations described above (Herold et al., 2009).

One important example for the application of specific T cell analyses are fungal infections, which are difficult to control and affect mainly immunocompromised patients. In fact, invasive fungal infections have become a major cause of infection-related mortality in immunocompromised patients, mainly caused by the two opportunistic fungi *Aspergillus, fumigatus* and *Candida albicans.* Initial, neutropenia and defects in the phagocyte cell function have been described as risk factors for developing invasive fungal infections but in the recent time it has been shown, that CD4⁺ T helper cells also play a critical role in the host defense against fungal pathogens.

In healthy individuals as well as in patients surviving invasive infection, antigen-specific proliferation of IFN-γ-producing T cells was detected upon stimulation with *Aspergillus* antigens (Hebart et al., 2002).

In addition, *Aspergillus* and *Candida* have been described to elicit distinct patterns of T_{H} cell cytokines, including T_{H}1 and T_{H}2 (Cenci et al., 1998a; Cenci et al., 1997; Hebart et al., 2002; Kurup et al., 2001), T_{H}17 (Bozza et al., 2009; Conti et al., 2009; Zhou et al., 2008), T_{H}22 (Liu et al., 2009) and even T_{reg} responses (Bozza et al., 2009). Several studies in mice and human suggest that a T_{H}1 response is correlated with antifungal protection whereas the production of T_{H}2 cytokines is linked to pathogenic fungal infection (Cenci et al., 1999; Cenci et al., 1998a; Cenci et al., 1998b; Cenci et al., 1997; Hebart et al., 2002; Perruccio et al., 2005). In addition, the recently identified T_{H}17 subset has also been implicated to play an important role in mucosal immunity against fungi, since studies in mice and humans demonstrated that the absence of IL-17 responses directly correlates with increased susceptibility to chronic and invasive *Candida* infections (Conti et al., 2009; Eyerich et al., 2008; Huang et al., 2004; Ma et al., 2008; Milner et al., 2008).

However, due to their very low frequencies comprehensive information about the frequency, phenotype and single antigen-specificity of fungi-reactive T cells in healthy donors and about disease associated changes are still missing. In particular direct *ex vivo* analyses of fungus-reactive T cells describing the total repertoire of T cells with specificity for fungi without prolonged in vitro manipulation is so far lacking. This is true for healthy donors and even more in immunocompromised persons. In the latter the detection can severely be affected by the fact that total T cell numbers may be drastically reduced or be deviated from standard values. Khanna et al (Blood 2009, 114: Abstract 1170) used the activation marker CD154 for identification of low frequency multiple novel Aspergillus fumigatus (AF) specific MHC class II epitopes after cultivation of cells in vitro for more than one week. In Khanna et al (Blood 2010, 116: Abstract 2326) they used the activation marker CD154 for selection of Aspergillus fumigatus specific T cells from stimulated peripheral blood mononuclear cells (PBMC) and co-cultured them with irradiated autologous PBMC for more than one week before analysis whereas AF-specific T cells were undetectable in PBMC.

In Jolink et al (Blood 2010, 116: Abstract 2332) peripheral blood mononuclear cells of healthy individuals were stimulated with overlapping 15mer peptides of the Aspergillus fumigatus proteins Crf1 and Catalase1. Directly after stimulation no antigen specific T cells could be detected, however after stimulation with the complete peptide pool, IL-2 and IL-15 for 7 days and subsequent restimulation with peptide pulsed autologous PBMC an increase of activated T cells could be detected in half of the healthy donors, based on IFNγ production, CD154 (CD40 ligand) and CD137 expression.

All of these technologies allow to analyse antigen-specific T Cells by flow-cytometry. However, the sensitivity limit of conventional flow-cytometry is about 0.1%. Many antigen-specific T cells in body fluids as e.g. blood samples or PBMC of patients or healthy donors are well below this limit and in addition for many situations it is important to further dissect the total pool of antigen-specific T cells into even smaller subpopulations which may have distinct functional characteristics, e.g cytokine producing cells. Changes within these subpopulations may have more diagnostic or prognostic value than the mere frequency of antigen-specific cells. Thus to detect small subpopulations with statistical significance, sufficiently high numbers of specific T cells have to be recorded, which necessitates to acquire high number of total T cells for each single measurement.

The sensitivity of flow-cytometry regarding rare cell detection is determined by the number of cells which can be acquired and the biological and methodological background, i.e. frequency of non-target cells expressing the marker and the frequency of false positive cells not expressing the marker. The latter depend largely on the quality of the sample and the specific features of target antigen.

To increase the limit of sensitivity, magnetic pre-enrichment of MHC-multimer-labelled T cells has been used (Sims et al. 2010 ; Vollers and Stem, 2008). In this way the few antigen-specific CD8 T cells in a large sample can be enriched by a rapid magnetic processing step, which is more or less not limited to a certain cell number, due to parallel processing of the cells on the magnetic column. In the second time limiting step, e.g. the flow-cytometric analysis, only the relatively few enriched cells have to be measured. In this way large cell numbers can be processed in a short period of time and this allows to sample sufficient numbers of target cells to analyse cells even at frequencies <10⁻⁶.

However due to the lack of suitable MHC class II tetramers this technology is not broadly applicable to CD4 T helper cells, which are of particular interest for autoimmune diseases or infections with extracellular pathogens, e.g. fungi or worms. Furthermore MHC multimers detect T cells only based on a certain affinity threshold of the antigen-receptor to the MHC/peptide-complex, which is somehow artificially determined by the specific multimeric features (avidity) of the particular multimer (Vollers and Stem, 2008). Thus T cells below this threshold might be lost although they may react to the naturally presented peptides on APC. Therefore the analysis of antigen-reactive T cells, as done via the use of "activation markers" is more reliable in terms of detection of cells, which have the functional capacity to play an active role in a particular immune response.

On the other hand the technologies for detection of antigen-specific T cells based on analysis of surface expressed activation markers such as CD154 or CD137 suffer from a relatively high frequency of "natural" background events, which are usually larger than 0.01-0.1%, which has therefore been considered to define the natural limit of sensitivity of this approach. This background is either induced already in vivo, i.e. small numbers of activated T cells in the blood circulation, or they become unspecifically activated upon *in vitro* culture.

Therefore, the enrichment prior to analysis of antigen-activated T cells expressing certain activation markers like CD154 or CD137 (WO2004/027428, EP appl. no. 10175578) in order to increase the limit of sensitivity has not been considered as a technological option and these technologies have so far not been used to analyse rare antigen-specific CD4 T cells like fungi-reactive T cells or small subsets thereof at frequencies below 0.1 %. In particular state-of-the-art was regarding these technologies as suitable for the analysis of antigen-specific memory T cells (> 0.01-0.1%) but not for the naive T cell repertoire where the single specificities have been calculated to occur at much lower frequencies (0.0001 %). Another technological difficulty was to simultaneously address activation markers on the surface, which is required for the enrichment, together with intracellular cytokines, an important functional parameter of T cells. To be able to detect intracellular cytokines the cells have to be incubated for prolonged time (several hours) with secretion inhibitors, e.g. monensin or brefeldin A, to achieve accumulation of the cytokines within the cell, followed by fixation. Secretion inhibitors at the same time block the export of the activation markers to the cell surface. This makes it difficult to achieve the combination of activation marker availability, e.g. for magnetic particle labelling and subsequent magnetic enrichment, with intracellular staining. In addition following the enrichment of small numbers of target cells further manipulations, e.g. fixation, intracellular cytokine staining are required which usually lead to cell loss, which affects the correct quantitation of target cell numbers and in addition reduces the sensitivity of the assay, due to lower target cells which can be analysed.

The object of the present invention is therefore to provide an improved method for quantitative and qualitative analysis of antigen-specific T cells or subpopulations thereof (referred to as "target" T cells) by the combined enrichment of the target cells followed by a second analysis step, without major cell loss and without the need for further in vitro manipulation of the cellular phenotype or function.

### Summary of the invention

The present invention solves the above technical problem by a method using magnetic cell separation using activation markers of antigen-specific T cells to increase the frequency of target T cells before further analysis of these cells, e.g. flow-cytometric analysis and the handling of the small cell numbers and associated cell loss was improved by manipulation of the cells directly on the columns used for enrichment.

It was surprising that we could establish a method for the specific enrichment of target T cells based on activation marker expression, e.g. CD154 expression, which allowed to separate rare antigen-specifically activated T cells from unspecific background. First, this was achieved by adjusting the conditions in a way that mainly brightly labelled T cells, which resemble those T cells which have been activated by antigen, are enriched versus low expressing "background" cells, resembling weakly activated, e.g. low affinity, T cells or T cells activated already in vivo at an earlier timepoint, i.e. residual activation marker expression or T cells activated by non TCR signals, i.e. bystander activation. Second, we have developed a new strategy to allow simultaneous detection of activation markers on the surface and accumulation of intracellular cytokines for their optimal detection by intracellular cytokine staining. This was achieved by subsequent stimulation with and without secretion inhibitors for optimized time periods. Another restriction of rare cell analysis is the cell loss during several processing steps, i.e. surface staining, fixation, intracellular staining. This cell loss severely affects the sensitivity of the method as well as the accuracy of quantitative and qualitative analysis. Therefore the invention includes staining of the cells following enrichment directly on the enrichment column, e.g. a magnetic column, especially MACS columns, which dramatically reduces cell loss, i.e. recovery of more target cells allowed us to accurately determine the target cell numbers as well as the analysis of phenotypic and functional subpopulations.

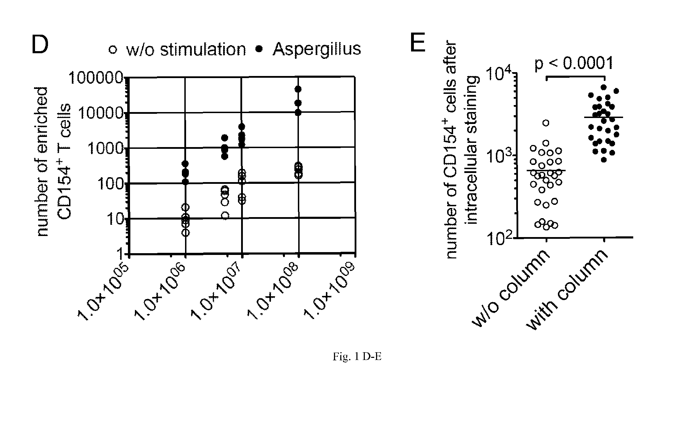

This new combined activation and labelling method allows to quantitatively select the target T cells on the basis of activation marker expression for their quantitation and simultaneous analysis of their functional and phenotypic properties. The invention allows to analyse large numbers of cells (at least up to 5x10¹⁰) for the presence of antigen-specific T cells and to characterise them phenotypically and functionally, e.g. for cytokine expression. The invention restricts the sensitivity of the analysis basically to the number of available input cells (see example 1, figure 1D). It was also surprising that even very small subpopulations of specific cytokine producing subsets or even naive T cells were detectable by the invention.

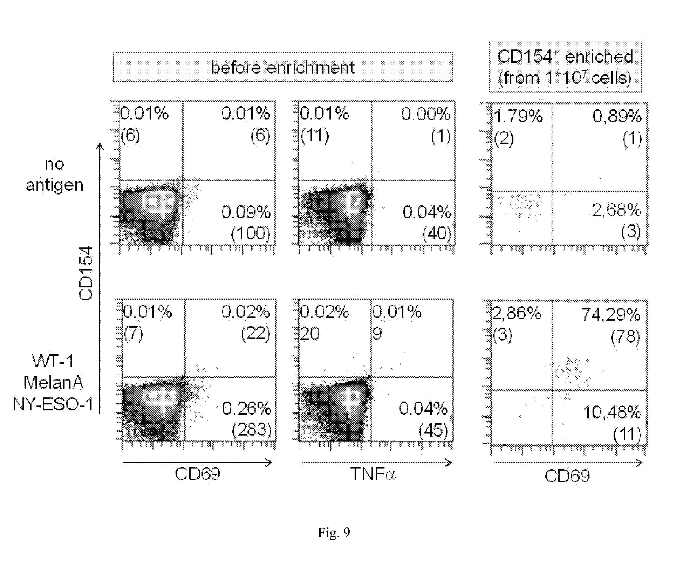

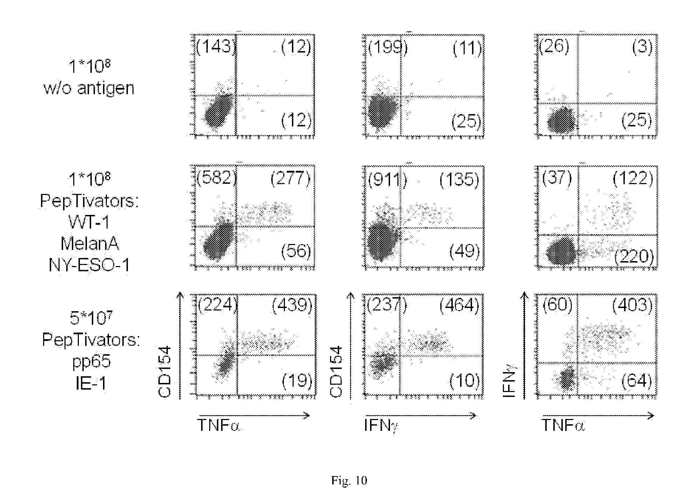

It was particularly surprising that we could analyse the natural repertoire of fungi reactive T cells in all healthy subjects tested as well as in patients with fungus associated diseases. The invention allowed us to define standard values for healthy subjects and define considerable changes associated with specific diseases, which might have prognostic or diagnostic values. These diseases associated changes occurred at the level of small subsets of antigen-specific T cells, producing certain cytokines and would not have been detectable by conventional means. Similarly we were able to identify and characterize tumor- or autoantigen-reactive CD4 T cells from healthy subjects as well as patients. These cells occurred in healthy subjects at frequencies as low as about 1 in 10⁵ (see figures 9 and 10).

### Short description of the figures

Figure 1. CD154-expression allows detection and enrichment of *Aspergillus-* and *Candida-*reactive CD4⁺ T cells from the blood of healthy donors.
Figure 2. Specific induction of CD154 following fungal lysate stimulation.
Figure 3. Phenotypic analysis of *Aspergillus-* versus *Candida*-reactive CD4⁺ T cells.
Figure 4. *Aspergillus-* and *Candida-*specific T cell produce different effector cytokines
Figure 5. Analysis of *Aspergillus*-reactive T cells in patients with hematologic malignancies
Figure 6. Increased frequencies and IL-22 production by Candida-reactive T cells in patients with Crohn's disease
Figure 7: In some patients with cystic fibrosis, the *Aspergillus-*reactive T cell response is biased towards a T_{H}2 cytokine pattern
Figure 8: Enrichment and characterization of antigen-specific regulatory T cells via CD137
Figure 9: Enrichment and characterisation of tumor/self antigen-specific T cells from 10⁷ peripheral blood cells from healthy donors using CD154
Figure 10: Enrichment and characterisation of tumor/self antigen-specific T cells from 10⁸ peripheral blood cells from healthy donors using CD154

### Detailed description of the invention

A method was surprisingly found for the qualitative and quantitative characterization of target T cells recognizing a specific antigen by the use of antigen-specific T cell activation markers, e.g. CD154 expression, directly from samples as e.g. body fluids or PBMC without the need of prior in vitro expansion of the cells.

The technologies for detection of antigen-specific T cells based on analysis of surface expressed activation markers such as CD154 or CD137 suffer from a relatively high frequency of "natural" background events, which are usually larger than 0.01-0.1%, which therefore defines the natural limit of sensitivity of this approach. Therefore, the pre-analysis enrichment of antigen-activated T cells expressing certain activation markers like CD154 or CD137 (WO2004/027428, EP appl. no. 10175578) has not been considered as a technological option and these technologies have so far not been used to analyse rare antigen-specific CD4 T cells like fungi-reactive T cells or small subsets thereof at frequencies below 0.1 %. Thus it was totally unexpected that these technologies are suitable for the analysis of the naive T cell repertoire where the single specificities have been calculated to occur at much lower frequencies (0.0001 %).

In addition the combination of enrichment and intracellular staining allows the further classification of cytokine producing subsets, which may present only 1% of the total antigen-specific T cell response and therefore would not be accessible without pre-enrichment step. To precisely define frequencies of small functional and phenotypic T cell subsets a sufficient number of antigen-specific target cells is necessary. Therefore we combined the high sensitivity of the enrichment with a minimal loss of target cells through characterization by performing all manipulations, e.g. permeabilisation, intracellular or surface staining procedures directly on the magnetic column. In this way, the sensitivity of the method is restricted to the number of available input cells and not affected by a loss of cells during washing and centrifugation steps, which are necessary for intracellular cytokine staining (see example 1, figure 1E).

The present invention relates to a method using magnetic cell separation using activation markers for selection of antigen-specific T cells to increase the frequency of target T cells and subsequent analysis of these cells, e.g. flow-cytometric analysis.

Information about the frequency phenotype and functional capabilities of the target T cells may have important diagnostic or prognostic value for infections or immune mediated diseases.

In a preferred embodiment of the invention the method comprises a method for sensitive quantitative and/or qualitative analysis of target T cells comprising the steps
a) enrichment of said cells from a mixture of said cells and other cells in a sample by the use of one or more activation markers expressed on antigen-activated T cells in a parallel cell sorting process and
b) analysis of the cells of step a).

In another preferred embodiment of the invention the parallel cell sorting process is a magnetic cell sorting process.

In another preferred embodiment of the invention the magnetic cell sorting process uses superparamagnetic beads and separation columns.

In another preferred embodiment of the invention manipulations of the target T cells following step a) are directly performed on the separation column.

In another preferred embodiment of the invention the activation markers are CD137 and/or CD154 and/or cytokines.

In another embodiment of the invention the method comprises a method for sensitive quantitative and/or qualitative analysis of target T cells comprising the steps
a) enrichment of said cells from a mixture of said cells and other cells in a sample by the use of the activation marker CD 154 expressed on antigen-activated T cells in a magnetic cell sorting process; and
b) analysis of the cells of step a), and
wherein the enrichment of the target T cells in said sample of step a) is after activation of the cells of the sample with an antigen and blocking or inhibiting the interaction between CD40 and CD154 by using a CD40/CD154 system inhibitor.

In another embodiment of the invention the method comprises a method for sensitive quantitative and/or qualitative analysis of target T cells comprising the steps
a) enrichment of said cells from a mixture of said cells and other cells in a sample by the use of the activation marker CD137 expressed on antigen-activated T cells in a magnetic cell sorting process; and
b) analysis of the cells of step a).

In another preferred embodiment of the invention the analysis of step b) is flow cytometry.

In another preferred embodiment of the invention the target T cells are specific for pathogens, tumor antigens, environmental antigens (allergens) or autoantigens.

In another preferred embodiment of the invention the target T cells are specific for fungi.

In another preferred embodiment of the invention the target T cells are specific for Aspergillus fumigatus and Candida albicans.

In another preferred embodiment of the invention the method comprises a method for diagnosing fungal infections in animals.

In another preferred embodiment of the invention the method comprises a method for diagnosing autoimmune diseases in animals.

In another preferred embodiment of the invention the method comprises a method for diagnosing allergies in animals.

In another preferred embodiment of the invention the method comprises a method for diagnosing tumor diseases in animals.

In another preferred embodiment of the invention the method comprises a method for monitoring or predict the success of vaccination with a certain antigen.

In another preferred embodiment of the invention, the antigen-specific T cells are obtained from a sample selected from the group of fresh blood, frozen cells, peripheral blood mononuclear cells (PBMC) and/or other body fluids.

In a further preferred embodiment of the method according to the invention, inflammatory, anti-inflammatory, regulatory and/or suppressive T lymphocytes are isolated and analysed. In some embodiments of the invention these cells may be used in cellular therapies for preventive or causal treatment of infectious, allergic, inflammatory, malignant and/or autoimmune diseases.

In another preferred embodiment of the invention the target T cells analysed within the mixture of said cells and other cells in the sample are below 0.1 % in frequency.

In another preferred embodiment of the invention the target T cells are CD4+ and/or CD8+ T cells.

In another preferred embodiment of the invention the target T cells are specific for fungi, especially pathogenic fungi. Preferentially, the target T cells specific for fungi are selected from the group of Candida, e.g. C. albicans, C.glabrata; Aspergillus, e.g. A. fumigatus, A. flavus, A. clavatus, A. terreus, A.niger, A.nidulans; Cryptococcus, e.g. C. neoformans, C. laurentii, C. albidus, C. gattii, Histoplasma capsulatum, Pneumocystis jirovecii (or Pneumocystis carinii), Stachybotrys chartarum. More preferentially, the target T cells specific for fungi are specific for Aspergillus fumigatus or Candida albicans.

In another preferred embodiment of the invention the target T cells are specific for tumor-antigens.

In another preferred embodiment of the invention the target T cells are specific for environmental antigens (allergens).

Lymphocytes and other leukocytes express a wide variety of molecules on their cell surface. Some appear for only a short time during particular phases of differentiation or following activation of the cells. Other molecules are constant and typical of the respective series of cells. Such antigens with constant expression can be utilized as markers for particular cell populations.

The term "target T cells" as used herein refers to rare antigen-specific T cells or subsets thereof in body fluids, as blood samples or PBMC of patients or healthy donors, which are below the sensitivity limit of a conventional flow-cytometry assay, which is often about 0.1-0.01 %. This detection limit may also refer to the frequency of certain subsets within the total population of specific T cells even if the total antigen-specific T cell frequency is above the limit, e.g. the frequency of a T cell subset producing a certain cytokine (see below). Therefore, for rare antigen-specific T cells recognizing a specific antigen, e.g. fungi-reactive T-cells comprehensive information about the frequency, phenotype and antigen-specificity of these cells in healthy donors and about disease associated changes are missing. For example it is currently unknown how big the total fungus-reactive T cell compartment actually is and how these cells are split of into different subsets (e.g. naive versus memory, expression of certain homing receptors, production of the crucial cytokines (e.g. IFN-gamma, IL-17, IL-22, IL-10). Using the invention we could show that these functional subsets can make up only 1-10% of the total reactive T cells (about 0.1% of all CD4 T cells), i.e. 0.001-0.01 % of the total CD4 T cells). It is also not known whether in fungus-associated diseases these numbers and/or frequencies are altered.

The term "activation marker" as used herein refers to proteins specifically expressed on the cell surface of T cells following their activation via their specific antigen-receptor, usually within 1-48 hours after activation. Activated T cells may comprise CD 4 T cells, CD8 T cells, naive T cells, memory T cells or regulatory T-cells. Such activation markers are, for example, CD154, CD137, secreted or membrane-anchored cytokines, e.g. IFN-gamma, IL-10, IL-2 TNF-alpha or "latent TGF-beta" (LAP), GARP (LRRC32), CD121a/b.

CD154 is a member of the TNF gene family and, inter alia, is expressed by various cells, particularly by T lymphocytes. CD154 is downregulated by the stimulated T lymphocytes one to three hours after activation. CD154 can be used to detect T lymphocytes independently of their functional potential. The use of CD154 in the detection and separation of T lymphocytes leads to reliable detection and isolation of T lymphocytes, possible independently of their function, i.e. all antigen-specific T lymphocytes in a sample can be determined and separated. There is also a therapeutic use of antigen-specific T cells following isolation based on their antigen-reactive CD154 expression, which can be induced after in vitro stimulation with defined antigens or antigen mixtures.

A method is disclosed in WO2004/027428 for the detection and/or isolation of antigen-specific T lymphocytes in a suspension following activation with an antigen, in which method the suspension is contacted with a CD40/CD154 system inhibitor, intra- and/or extracellular determination of CD154 is effected, and the cells having CD154 are detected and/or isolated. Addition of a CD40/CD154 system inhibitor impairs or inhibits the interaction and signalling between CD40 and CD 154. CD40/CD 154 system inhibitors can be any of molecules or even physical exposures capable of blocking or inhibiting the interaction between CD40 and CD154. Accordingly, the inhibiting agent can be an antibody, e.g. one directed against CD40, a molecule, a cesium or lithium ion having an effect on the interaction between CD40 and CD154. Of course, said agent can also be a substance inhibiting the secretion or endocytosis in the cell, such as brefeldin A (Bref-A). Bref-A inhibits the Golgi apparatus and the secretion of a variety of cytokines. These substances ensure that CD40, CD154, the interaction between the two of them, or the CD40/CD154 system are modified in such a way that CD154 either is no longer downregulated and/or degraded on the cell surface, or, provided it is still within the cell, no longer transported therein. Such interruption of the transport within the cell prevents degradation of CD154. Consequently, CD154 is stabilized inside or outside the cell as an external receptor, thereby allowing detection and subsequent isolation using detection methods well-known to those skilled in the art.

EP appl. no. 10175578 disclosed that the use of CD154 as a negative selection marker greatly increases the window of time for the use of the marker CD137 for the positive selection of regulatory T cells (Treg) and that 2 to 24 hours after activation, regulatory T-cells can be isolated with great purity and yield. Therefore it is possible with the present invention to analyse rare antigen-specific Treg cells using the combination of activation markers CD137 and CD154.

For selection in principle any "parallel" sorting technology can be used. Parallel refers to methods where the selection of the target cells takes place in a single separation step from the bulk population of cells. This includes for example affinity chromatography or any other antibody-dependent separation technique known in the art, which allows parallel processing. Any ligand-dependent separation technique known in the art may be used in conjunction with both positive and negative separation techniques that rely on the physical properties of the cells. In contrast to parallel sorting "single cell technologies" exist, where cells are analysed subsequently one after the other and are very time consuming especially for large cell numbers.

An especially potent parallel sorting technology is magnetic cell sorting. The term "magnetic cell sorting" or "magnetic cell sorting process" is used herein to refer to procedures for cell separation (cell sorting) including, but are not limited to, magnetic separation using antibodies linked to colloidal magnetic particles or micron-sized magnetic particles (e.g. 1-10 µm). Methods to separate cells magnetically are commercially available e.g. from Invitrogen, Stem cell Technologies, in Cellpro, Seattle or Advanced Magnetics, Boston. For example, autologous monoclonal antibodies can be directly coupled to magnetic polystyrene particles like Dynal M 450 or similar magnetic particles and used e.g. for cell separation. Alternatively, antibodies can be biotinylated or conjugated with digoxigenin and used in conjunction with avidin or anti-digoxigenin coated affinity columns. In a preferred embodiment however, monoclonal antibodies are used in conjunction with colloidal superparamagnetic microparticles having an organic coating by e.g. polysaccharides (Miltenyi et al., 1990). These particles can be used having a size of 10 to 200 nm, preferably between 40 and 100 nm, and can be either directly conjugated to autologous antibodies or used in combination with anti-immunoglobulin, avidin or antihapten-specific microbeads. Polysaccharide-coated superparamagnetic particles are commercially available from Miltenyi Biotec GmbH, Germany.

The term "manipulation" of cells as used herein refers to manipulations or modification of cells, e.g. permeabilisation, intracellular or surface staining procedures. The manipulations can be performed directly on the magnetic columns in parallel to or after the enrichment step of the present inventive method but prior to the cell analysis step. Alternatively manipulations of cells, e.g. fixation of cells can be performed in suspension. If separation of target T cell is combined with an intracellular staining, surface staining can be performed on the first column, followed by fixation and intracellular cytokine staining on the second column.

As used herein, the term "antibody" is intended to include polyclonal and monoclonal antibodies, chimeric antibodies, haptens and antibody fragments, and molecules which are antibody equivalents in that they specifically bind to an epitope on the product antigen. The term "antibody" includes polyclonal and monoclonal antibodies of any isotype (IgA, IgG, IgE, IgD, IgM), or an antigen-binding portion thereof, including, but not limited to, F(ab) and Fv fragments such as sc Fv, single chain antibodies, chimeric antibodies, humanized antibodies, and a Fab expression library.

The cells can be analysed (characterized) after magnetic pre-enrichment according to all methods known to the person skilled in the art. Preferred for the characterization of cells are in particular cell sorting (e.g. further magnetic cell sorting (MACS)), fluorescence activated cell sorting (FACS), ELISA, PCR and/or all fluorescence microscopes known in the art. Particularly preferred is the use of flow-cytometry (FACS). For flow-cytometry, e.g. FACS (fluorescence activated cell sorting), antibodies are used that may be coupled with fluorescence markers that are known to a person of skill in the art, like FITC, phycoerythrin (PE), allophycocyanin (APC), cascade yellow and peridinin chlorophyll protein (PerCP).

The target T cells are directly identified and/or separated or isolated from blood, peripheral mononuclear blood cells (PBMC), body tissue or cells from tissue fluids.

The cells are normally identified and/or separated from cell samples from mammals (Mamalia), but especially from humans and preferably from test subject and/or patients. The cells may be derived for example from blood samples which comprise immune cells (peritoneal, cerebral-spinal, pleural and/or synovial body fluids, lavage fluids from hollow organs (airways, lungs), homogenates and aspirates of lymph nodes, spleen, tonsils and/or other lymphatic tissue). The target T cells may also be obtained from animal tissues (e.g. from the spleen or the lymph nodes of an animal).

The separated activated antigen-specific T cells can be used before and/or after cloning and/or growing and/or concentrated in cell mixtures in and/or as pharmaceutical composition in the therapy or prevention of diseases. It is additionally possible for the coding gene sequences of the TCR (T-cell receptor) to be isolated from the separated antigen-specific T cells and be used for further therapeutic purposes such as, for example, for cellular therapies. It is additionally possible to employ the activated antigen-specific T cells in the form mentioned in further investigations and/or analyses. The pharmaceutical composition can be used for the treatment and/or prevention of diseases in mammals, possibly including administration of a pharmaceutically effective amount of the pharmaceutical composition to the mammal.

The disease may be any disease, which can be treated and/or prevented through the presence of a separated cell and/or through increasing the concentration of the relevant cells in/at the relevant place, or in whole mammalian subjects and/or patients. The cell may be for example an activated rare antigen-specific cell, and the treated and/or preventively treated disease may be an autoimmune disease, an infectious disease, an allergy, transplant versus host disease (or allogeneic transplant rejection) and/or any other disease initiated by hypersensitivity. Diseases for which the use set forth in the invention is particularly suitable are those arising through and/or during a lack of regulation of the immune response. These diseases may be transplant rejections, allergic conditions, certain infectious diseases, tumors and/or autoimmune diseases.

The invention may be used for obtaining information with prognostic or diagnostic value for certain diseases. In principle changes in the absolute number, the frequency or the phenotype or function of antigen-specific T cells during the course of the disease or in a patient relative to a control cohort may provide this kind of information, since a certain change may be associated with improved or diminished protection or might be indicative for an active disease, i.e. fungus infection. To obtain this information the antigen-specific T cells e.g. from peripheral blood of patients and/or suitable controls can be enriched and analysed as described in the invention and compared to standard values from healthy subjects or other control groups and the results may be used for diagnostic or prognostic purposes. This may comprise the following examples but may not be limited to those:
1. Invasive Aspergillosis (IA): IA is difficult to diagnose timely and the information abut an altered frequency of Aspergillus reactive T cells or an altered cytokine pattern might be indicative for an ongoing IA or a better or worse chance to clear the infection. Similarly assessing the donors' Aspergillus-specific repertoire might be indicative for the chance to develop Aspergillus infection following hematopoietic stem cell transplantation.
2. Colitis: Candida is residing in the gut of healthy individuums. In inflammatory bowel disease the immune response against gut residing microbiota or pathogens might be altered and could be indicative of disease activity or may be a direct link to the pathogenicity, i.e. inflammatory T cell reactions against the gut residing organisms supports the inflammation. E.g. the data provided herein indicate that the response to Candida is significantly altered in patients with Morbus Crohn.
3. Cystic fibrosis: Patients with cystic fibrosis frequently suffer from fungal infections, especially Aspergillus, in the lung accompanied by severe allergic reactions. The invention might be used to identify the patients with acute fungal infections or allergies against a certain fungus by analysing the fungus specific T cells response and identify a characteristic cytokine pattern, e.g. Th2 cytokine production which is usually not observed in healthy controls
4. Allergies: In general allergies might be sensitively diagnosed by analysing the cytokine pattern of allergen reactive T cells and/or the frequency/number of allergen reactive T cells.
5. Autoimmunity: Measuring the frequency/phenotype of autoreactive T cells, e.g. effector versus regulatory-type T cells, might be indicative for a certain disease activity, disease course or severity.
6. Analysing the naïve repertoire for prognosis for the development of certain infections, tumors or allergies: the naïve repertoire against certain infectious agents, tumors or autoantigens differs in each individuum. Obtaining information about the frequency and phenotype of the specific T cells even in the non-diseased state might be of prognostic value how the immune response develops, e.g. success of vaccination against a certain tumor/allergen/auto- antigen may depend on the frequency of T cells specific against this antigen in the naïve compartment. The invention may be used to quantify and characterize the few reactive T cells against a defined antigen or specific epitope thereof and may be used for rapid screening of the most effective therapeutic or prophylactic treatment.

The invention can also be used to separate and analyse T cells recognizing a specific single protein or peptide from Aspergillus or other pathogens resulting in a characterization of potential reactive epitopes in donors. For this goal the sample, e.g. PBMC or other body fluids, is stimulated with a single protein or peptide instead of a lysate prior to enrichment and analysis according to the present invention.

### References

Alanio, C., Lemaitre, F., Law, H.K., Hasan, M., and Albert, M.L. Enumeration of human antigen-specific naive CD8+ T cells reveals conserved precursor frequencies. Blood 115, 3718-3725.
Altman, J.D., and Davis, M.M. (2003). MHC-peptide tetramers to visualize antigen-specific T cells. Curr Protoc Immunol Chapter 17, Unit 17 13.
Bozza, S., Clavaud, C., Giovannini, G., Fontaine, T., Beauvais, A., Sarfati, J., D'Angelo, C., Perruccio, K., Bonifazi, P., Zagarella, S., et al. (2009). Immune sensing of Aspergillus fumigatus proteins, glycolipids, and polysaccharides and the impact on Th immunity and vaccination. J Immunol 183, 2407-2414.
Campbell, J.D., Foerster, A., Lasmanowicz, V., Niemoller, M., Scheffold, A., Fahrendorff, M., Rauser, G., Assenmacher, M., and Richter (2011). A. Rapid detection, enrichment and propagation of specific T cell subsets based on cytokine secretion. Clin Exp Immunol 163, 1-10.
Cenci, E., Mencacci, A., Del Sero, G., Bacci, A., Montagnoli, C., d'Ostiani, C.F., Mosci, P., Bachmann, M., Bistoni, F., Kopf, M., and Romani, L. (1999). Interleukin-4 causes susceptibility to invasive pulmonary aspergillosis through suppression of protective type I responses. J Infect Dis 180, 1957-1968.
Cenci, E., Mencacci, A., Fe d'Ostiani, C., Del Sero, G., Mosci, P., Montagnoli, C., Bacci, A., and Romani, L. (1998a). Cytokine- and T helper-dependent lung mucosal immunity in mice with invasive pulmonary aspergillosis. J Infect Dis 178, 1750-1760.
Cenci, E., Mencacci, A., Fe d'Ostiani, C., Montagnoli, C., Bacci, A., Del Sero, G., Perito, S., Bistoni, F., and Romani, L. (1998b). Cytokine- and T-helper-dependent immunity in murine aspergillosis. Res Immunol 149, 445-454; discussion 504-445.
Cenci, E., Perito, S., Enssle, K.H., Mosci, P., Latge, J.P., Romani, L., and Bistoni, F. (1997). Th1 and Th2 cytokines in mice with invasive aspergillosis. Infect Immun 65, 564-570.
Chai, L.Y., van de Veerdonk, F., Marijnissen, R.J., Cheng, S.C., Khoo, A.L., Hectors, M., Lagrou, K., Vonk, A.G., Maertens, J., Joosten, L.A., et al. Anti-Aspergillus human host defence relies on type 1 T helper (Th1), rather than type 17 T helper (Th17), cellular immunity. Immunology 130, 46-54.
Chattopadhyay, P.K., Yu, J., and Roederer, M. (2005). A live-cell assay to detect antigen-specific CD4+ T cells with diverse cytokine profiles. Nat Med 11, 1113-1117.
Chaudhary, N., Staab, J.F., and Marr, K.A. Healthy human T-Cell Responses to Aspergillus fumigatus antigens. PLoS One 5, e9036.
Conti, H.R., Shen, F., Nayyar, N., Stocum, E., Sun, J.N., Lindemann, M.J., Ho, A.W., Hai, J.H., Yu, J.J., Jung, J.W., et al. (2009). Th17 cells and IL-17 receptor signaling are essential for mucosal host defense against oral candidiasis. J Exp Med 206, 299-311.
Day, C.L., Seth, N.P., Lucas, M., Appel, H., Gauthier, L., Lauer, G.M., Robbins, G.K., Szczepiorkowski, Z.M., Casson, D.R., Chung, R.T., et al. (2003). Ex vivo analysis of human memory CD4 T cells specific for hepatitis C virus using MHC class II tetramers. J Clin Invest 112, 831-842.
Eyerich, K., Foerster, S., Rombold, S., Seidl, H.P., Behrendt, H., Hofmann, H., Ring, J., and Traidl-Hoffmann, C. (2008). Patients with chronic mucocutaneous candidiasis exhibit reduced production of Th17-associated cytokines IL-17 and IL-22. J Invest Dermatol 128, 2640-2645.
Frentsch, M., Arbach, O., Kirchhoff, D., Moewes, B., Worm, M., Rothe, M., Scheffold, A., and Thiel, A. (2005). Direct access to CD4+ T cells specific for defined antigens according to CD154 expression. Nat Med 11, 1118-1124.
Hebart, H., Bollinger, C., Fisch, P., Sarfati, J., Meisner, C., Baur, M., Loeffler, J., Monod, M., Latge, J.P., and Einsele, H. (2002). Analysis of T-cell responses to Aspergillus fumigatus antigens in healthy individuals and patients with hematologic malignancies. Blood 100, 4521-4528.
Herold, K.C., Brooks-Worrell, B., Palmer, J., Dosch, H.M., Peakman, M., Gottlieb, P., Reijonen, H., Arif, S., Spain, L.M., Thompson, C., and Lachin, J.M. (2009). Validity and reproducibility of measurement of islet autoreactivity by T-cell assays in subjects with early type 1 diabetes. Diabetes 58, 2588-2595.
Huang, W., Na, L., Fidel, P.L., and Schwarzenberger, P. (2004). Requirement of interleukin-17A for systemic anti-Candida albicans host defense in mice. J Infect Dis 190, 624-631.
Kirchhoff, D., Frentsch, M., Leclerk, P., Bumann, D., Rausch, S., Hartmann, S., Thiel, A., and Scheffold, A. (2007). Identification and isolation of murine antigen-reactive T cells according to CD154 expression. Eur J Immunol 37, 2370-2377.
Kurup, V.P., Xia, J.Q., Crameri, R., Rickaby, D.A., Choi, H.Y., Fluckiger, S., Blaser, K., Dawson, C.A., and Kelly, K.J. (2001). Purified recombinant A. fumigatus allergens induce different responses in mice. Clin Immunol 98, 327-336.
Liu, Y., Yang, B., Zhou, M., Li, L., Zhou, H., Zhang, J., Chen, H., and Wu, C. (2009). Memory IL-22-producing CD4+ T cells specific for Candida albicans are present in humans. Eur J Immunol 39, 1472-1479.
Ma, C.S., Chew, G.Y., Simpson, N., Priyadarshi, A., Wong, M., Grimbacher, B., Fulcher, D.A., Tangye, S.G., and Cook, M.C. (2008). Deficiency of Th17 cells in hyper IgE syndrome due to mutations in STAT3. J Exp Med 205, 1551-1557.
Milner, J.D., Brenchley, J.M., Laurence, A., Freeman, A.F., Hill, B.J., Elias, K.M., Kanno, Y., Spalding, C., Elloumi, H.Z., Paulson, M.L., et al. (2008). Impaired T(H)17 cell differentiation in subjects with autosomal dominant hyper-IgE syndrome. Nature 452, 773-776.
Miltenyi, S., Muller, W., Weichel, W., and Radbruch, A. (1990). High gradient magnetic cell separation with MACS. Cytometry 11, 231-238.
Moon, J.J., Chu, H.H., Pepper, M., McSorley, S.J., Jameson, S.C., Kedl, R.M., and Jenkins, M.K. (2007). Naive CD4(+) T cell frequency varies for different epitopes and predicts repertoire diversity and response magnitude. Immunity 27, 203-213.
Nepom, G.T. (2005). Tetramer analysis of human autoreactive CD4-positive T cells. Adv Immunol 88, 51-71.
Perruccio, K., Tosti, A., Burchielli, E., Topini, F., Ruggeri, L., Carotti, A., Capanni, M., Urbani, E., Mancusi, A., Aversa, F., et al. (2005). Transferring functional immune responses to pathogens after haploidentical hematopoietic transplantation. Blood 106, 4397-4406.
Potenza, L., Barozzi, P., Vallerini, D., Bosco, R., Quadrelli, C., Mediani, L., Morselli, M., Forghieri, F., Volzone, F., Codeluppi, M., et al. (2007). Diagnosis of invasive aspergillosis by tracking Aspergillus-specific T cells in hematologic patients with pulmonary infiltrates. Leukemia 21, 578-581.
Sims, S., Willberg, C., and Klenerman, P. MHC-peptide tetramers for the analysis of antigen-specific T cells. Expert Rev Vaccines 9, 765-774.
Thiel, A., Scheffold, A., and Radbruch, A. (2004). Antigen-specific cytometry--new tools arrived! Clin Immunol 111, 155-161.
Vollers, S.S., and Stern, L.J. (2008). Class II major histocompatibility complex tetramer staining: progress, problems, and prospects. Immunology 123, 305-313.
Warris, A., Netea, M.G., Verweij, P.E., Gaustad, P., Kullberg, B.J., Weemaes, C.M., and Abrahamsen, T.G. (2005). Cytokine responses and regulation of interferon-gamma release by human mononuclear cells to Aspergillus fumigatus and other filamentous fungi. Med Mycol 43, 613-621.
Wehler, T.C., Karg, M., Distler, E., Konur, A., Nonn, M., Meyer, R.G., Huber, C., Hartwig, U.F., and Herr, W. (2008). Rapid identification and sorting of viable virus-reactive CD4(+) and CD8(+) T cells based on antigen-triggered CD137 expression. J Immunol Methods 339, 23-37.
Wolfl, M., Kuball, J., Eyrich, M., Schlegel, P.G., and Greenberg, P.D. (2008). Use of CD137 to study the full repertoire of CD8+ T cells without the need to know epitope specificities. Cytometry A 73, 1043-1049.
Wolfl, M., Kuball, J., Ho, W.Y., Nguyen, H., Manley, T.J., Bleakley, M., and Greenberg, P.D. (2007). Activation-induced expression of CD137 permits detection, isolation, and expansion of the full repertoire of CD8+ T cells responding to antigen without requiring knowledge of epitope specificities. Blood 110, 201-210.
Zhou, M., Yang, B., Ma, R., and Wu, C. (2008). Memory Th-17 cells specific for C. albicans are persistent in human peripheral blood. Immunol Lett 118, 72-81.

Without intending to be limiting, the invention will be illustrated with reference to the following examples.

### Examples

### Example 1: Aspergillus- and Candida-reactive CD4⁺ T cells can be detected with high sensitivity in the blood of healthy donors using CD154 enrichment

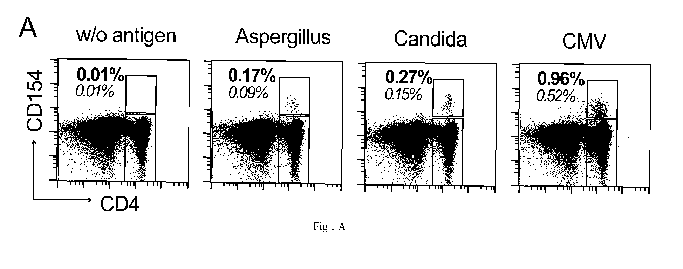

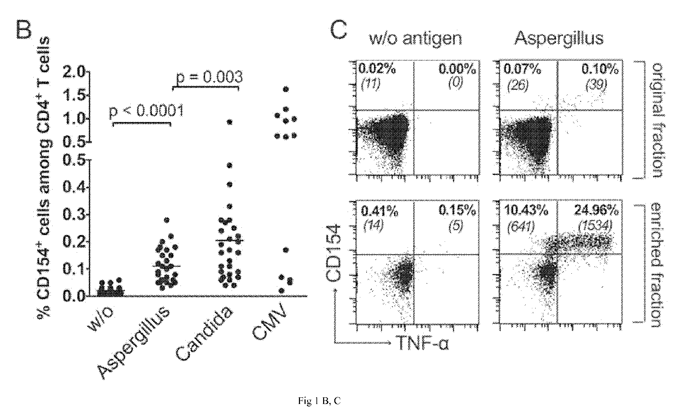

CD154 expression allows the detection of the entire pool of antigen reactive CD4⁺ T cells, without any restriction to a certain subset producing particular cytokines. We therefore used this marker to perform a comprehensive characterization of T cells specific for the two important opportunistic fungal pathogens *Aspergillus fumigatus* and *Candida albicans.* Human peripheral blood mononuclear cells (PBMCs) were stimulated *in vitro* with lysates of the respective antigens. As shown in figure 1A and B, *Aspergillus* and *Candida* stimulation induced a small, but clear cut population of CD154 expressing CD4⁺ T cells in all tested donors (p < 0.0001 compared to non-stimulated control), with a mean of 0.1% and 0.2%, respectively. By contrast, stimulation with CMV-lysate as a control antigen, induced either high frequencies of CD154⁺ cells in serum-positive donors or background frequencies in serum negative donors (Figure 1B).

To increase the limit of sensitivity and to enable the analysis of a significant number of fungus-reactive T cells, we made use of the possibility to pre-enrich reactive cells via magnetic selection of CD154⁺ T cells before analysis. In this way, antigen specific T cells from 10⁷-10¹⁰ PBMC can be rapidly analysed. As shown in figure 1C, CD 154 enrichment resulted in a sizeable population of *Aspergillus-*reactive cells, allowing the further characterization and detection of even small subpopulations, e.g. producers of certain cytokines.

To determine the sensitivity of our method, we measured the number of enriched CD154⁺ cells with and without antigen stimulation, using different starting cell numbers (Figure 1 D). With 10⁸ PBMCs about 100-200 CD154⁺ cells were enriched without antigen stimulation. At lower starting cell frequencies (10⁶ to 10⁷) the background numbers ranged between 5-100. These data show, that the sensitivity of the method depends on the number of input cells with a detection limit of about 10⁻⁵-10⁻⁶ for 10⁷-10⁸ starting cells.

For an intracellular cytokine staining several washing and centrifugation steps are essential, which result in a decrease of target cells. This target cell loss is especially high in situations where only few target cells can be recovered. Thus the general sensitivity of the method is diminished and furthermore the quantitative determination of target cell numbers and the characterisation of small subsets within the target cells is strongly affected. Therefore we developed a method to combine the high sensitivity of the CD154-enrichment with a minimal loss of cells during further characterization by performing all staining procedures directly on a MACS column. In this way, the number of available cells for analysis is strongly increased, when compared to the standard procedure without column staining, which allows more accurate determination of absolute cell numbers as well as a more precise definition and detection of functionally or phenotypically defined T cell subsets within the target population (Figure 1E).

### Detailed description of the Figure 1

PBMCs were stimulated for with the indicated lysates and analyzed for CD154 expression. CD154⁺ cells among CD4⁺ (bold) or among total lymphocytes (italics) are shown of (A) one representative donor and (B) of several donors *(Aspergillus, Candida* n=28, CMV n=13) with indicated mean. *p* values were determined using paired Student's t-test. (C) Magnetically enrichment of CD154⁺ T cells followed by intracellular cytokine staining. CD154 expression was assessed among CD4⁺ T cells after stimulation (upper plots) and after MACS sorting (lower plots). Numbers in brackets indicate cell count and the total numbers of cells obtained from 10⁷ PBMCs (lower plots). (D) Determination of the detection limit of the assay. The total number of enriched CD154⁺ cells from different starting cell numbers of *Aspergillus-* or non-stimulated PBMCs is shown (n = 5). (E) CD154⁺ cell numbers out of 10⁷ PBMCs after *Aspergillus* stimulation performing intracellular cytokine staining either with MACS column or after separation (n = 30). Significance was determined using unpaired Student's t-test.

### Preparation of fungal extracts

The *A. fumigatus* strain ATCC 46645 (LGC Standars, Wesel, Germany) was used for preparation of crude protein extracts from mycelium. Conidia were inoculated at a final concentration of 2·10⁶ spores/ml in YPD medium and shaken at 37 °C with 200 rpm. After 20 hours incubation, mycelium was recovered by filtration, washed with water and stored at -70 °C. Frozen mycelium was resuspended in saline (0.9% [w/v] NaCl) and disrupted in a microdismembrator (Sartorius, Göttingen, Germany) at 200 rpm for 10 min using 0.5 ∅ mm glass beads. The extract was resuspended in PBS (2mM MgCl₂) and centrifuged for 20 min at 20000 x g. Supernatants were stored in aliquots at -20 °C until use.

### Stimulation of human PBMCs and isolation of antigen-specific T cells

Buffy coats from healthy donors were obtained from the university hospital in Dortmund. Peripheral blood samples were obtained from healthy volunteers and 31 patients with Crohn's disease, 9 patients with ulcerative colitis, 8 patients with cystic fibrosis and 17 patients with hematologic malignancies. Peripheral blood mononuclear cells (PBMCs) were separated by use of Ficoll-Hypaque (GE-Healthcare Bio-Science) density gradient centrifugation, washed twice in CliniMACS buffer (Miltenyi Biotec) and resuspended in RPMI-1640 (Miltenyi Biotec), supplemented with 5% (v/v) inactivated human AB-serum (Lonza) and 2mM L-glutamine (PAA Laboratories). Stimulations in all experiments were performed with the same concentrations of antigens: *A. fumigatus, A.terreus, A.nidulans, A.niger, R.oryzae, S.Scedosporium, A.corybifera* (40µg/ml), C. *albicans* (20µg/ml; Greer Laboratories), CMV-lysate (10µg/ml; Siemens), E.coli recombinant proteins (50µg/ml), SEB (1µg/ml; Sigma Aldrich) in the presence of 1µg/ml CD28 functional grade pure antibody (Miltenyi Biotec). Each setting contained a non-stimulated sample as negative control.

For separation of antigen-specific T cells, 1x10⁷ PBMCs/ml were stimulated for 7 hours with the different antigens at 37°C in a 5% (v/v) CO₂ atmosphere. To prevent down-regulation of CD 154 expression, 1µg/ml CD40 functional grade pure antibody (Miltenyi Biotec) was added. If separation of antigen-specific T cell was combined with an intracellular staining, 1µg/ml Brefeldin A (Sigma Aldrich) was added for the last 2 hours of stimulation.

After stimulation, cells were separated using the CD154 MicroBead Kit (Miltenyi Biotec). In brief, cells were indirectly magnetically labelled with CD154-Biotin and anti-Biotin microbeads in a titer of 1:10 and enriched by two MS MACS columns (Miltenyi Biotec). For characterization of phenotypic markers, surface staining was performed onto the second column in a total staining volume of 100µl. If separation of antigen-specific T cell was combined with an intracellular staining, surface staining was performed on the first column, followed by fixation and intracellular cytokine staining on the second column. Cells were eluted from the first column with 500µl of buffer and 500µl InsideFix (Inside stain Kit; Miltenyi Biotec) was added for 20min at room temperature. The fixed cell suspension was applied onto a second MS MACS column, washed with InsidePerm (Inside stain Kit; Miltenyi Biotec) and stained intracellular in a total volume of 100µl.

### Example 2: CD154 up-regulation following stimulation with fungal lysates is antigen-specific

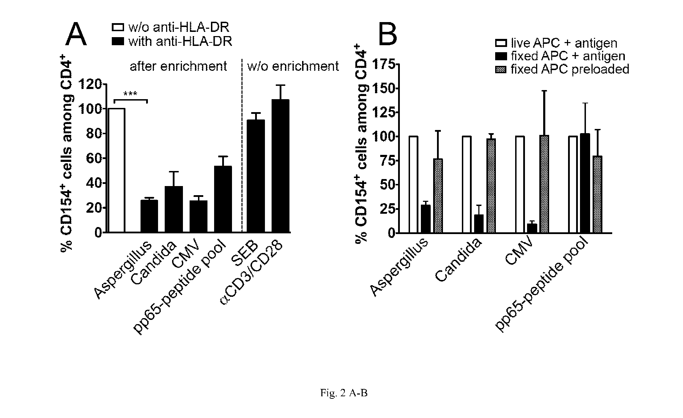

To exclude non-specific stimulation by fungal components potentially present in the total fungi lysates we blocked the T cell activation by addition of an HLA-DR-neutralising antibody. This antibody blocked the CD4⁺ T cell responses against *Aspergillus-, Candida-*and CMV-lysates as well as against a peptide pool of CMV pp65 but neither T cell activation by SEB and antiCD3/CD28 (Figure 2A) nor the activation of CD8⁺ T cells (not depicted). Similarly, the CD4⁺ T cell responses against the lysates but not against the pp65 peptide pool were prevented when APCs were fixed with formaldehyde before incubation with the antigens (Figure 2B). These data clearly show that T cell activation occurs through an MHC class II- and antigen processing-dependent mechanism.

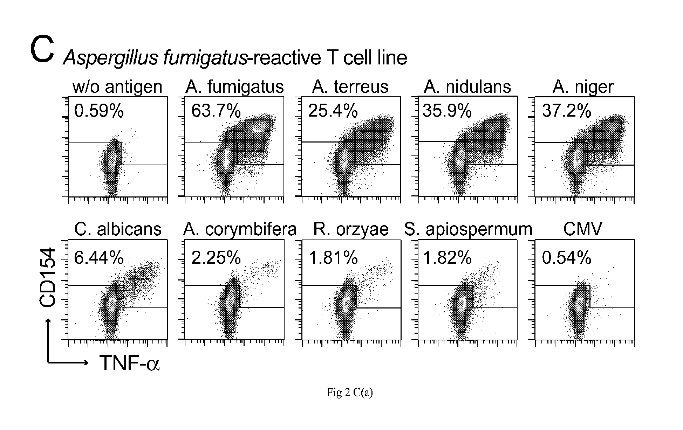

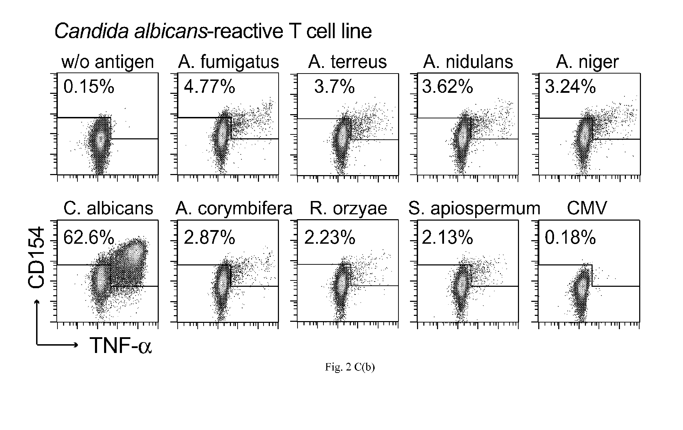

To further confirm the specificity of the fungus activated CD154⁺ cells, we used the CD154 enrichment for the rapid generation of polyclonal *Aspergillus-* and *Candida-*specific T cell lines. Following stimulation with *Aspergillus-* or *Candida*-lysate*,* the CD154⁺ T cells were magnetically isolated and further expanded in the presence of autologous feeder cells and IL-2. To determine frequencies and functionalities, the T cell lines were re-stimulated with autologous APCs and the indicated antigens. The expanded T cells show high reactivity towards the inducing antigen (> 60%), as shown by up-regulation of CD 154 and expression of cytokines (Figure 2C). Furthermore we observed high cross-reactivity of *Aspergillus fumigatus-*specific T cell lines with *Aspergillus terreus, Aspergillus nidulans* and *Aspergillus niger.* A small proportion of *Aspergillus-*reactive T cells also reacted to *Candida albicans* antigens and *vice versa,* as well as other fungi (< 3%), such as *Absidia corymbifera, Rhizopus oryzae* or *Scedosporium apiospermum.* In contrast, the reactivity against CMV lysate was at background level. These data suggest, that there is limited overlap between antigens from various fungal species and confirm the high-specificity of the CD 154 enrichment assay, suggesting that the frequency of antigen-specific T cells can be determined with high sensitivity.

### Detailed description of the figure 2

(A) Inhibition of the CD154 induction by an antiHLA-DR blocking antibody. PBMCs of pp65-tetramer positive donors were stimulated with the indicated antigens in the presence or absence of antiHLA-DR mAb and the number of CD154⁺ T cells after enrichment (left) or the frequencies of CD154⁺ T cells without enrichment (right) were analyzed. Bars show mean percentages and SEM from 4 donors referring to samples without antiHLA-DR. Two independent experiments were performed. (B) CD3⁺ T cells and CD3⁻ APCs were separated from PBMCs of pp65 tetramer positive donors. Stimulation was performed under the indicated conditions and the number of CD154⁺ T cells after enrichment was analyzed. Bars indicate mean percentages and SEM of CD154⁺ cells referring to live APCs + antigen stimulated samples from 3 different donors. (C) PBMCs of CMV sero-positive donors were stimulated with *Aspergillus-* or *Candida-*lysate*.* Antigen-reactive CD154⁺ cells were isolated and subsequently expanded for 14 days with IL-2 and autologous feeder cells. Expanded cell lines were re-stimulated in presence of autologous APCs with and without antigens as indicated, and reactive CD4⁺ T cells were determined by CD154 and TNF-α expression. Data of one representative donor out of 7 with percentage of reactive cells among CD4⁺ are shown.

### HLA-DR blocking experiments and fixation of APCs

For inhibition experiments, 1x10⁷ PBMCs from CMV sero-positive donors were stimulated with *A. fumigatus-,* C. *albicans-,* CMV-lysate as described above, or CMV pp65 PepTivator (0.6nmol/ml; Miltenyi Biotec), SEB (1µg/ml; Sigma Aldrich), anti-CD3/CD28 (Miltenyi Biotec) in presence or absence of an HLA-DR specific mAb (100 µg/ml clone AC 122; Miltenyi Biotec). CD154⁺ T cells were isolated, as described above, using the CD154⁺ MicroBead Kit (Miltenyi Biotec) and the number of CD154⁺ cells after enrichment or the frequencies of CD154⁺ cells after stimulation (SEB, antiCD3/CD28) was analyzed by flow cytometry.

For antigen-processing experiments, T cells and APCs were separated from PBMCs using an untouched isolation with the pan T cell isolation Kit II (Miltenyi Biotec) and depletion of CD3⁺ T cells via anti-CD3 microbeads (Miltenyi Biotec), respectively. Both fractions reached a purity > 99%. CD3-depleted APCs were either loaded over night with different antigens or left unloaded, respectively. Loaded and unloaded APCs were fixed in 0.18% (v/v) paraformaldehyde in CliniMACS buffer (Miltenyi Biotec) for 5 min at RT and then washed extensively in RPMI-1640 medium. 5x10⁶ APCs were used for the stimulation of 5x10⁶ T cells. After stimulation, CD154 expressing cells were separated, as described above, and analyzed by flow cytometry. The number of enriched CD154⁺ cells was compared with the control using non-fixed APCs plus antigens for stimulation.

### Expansion and re-stimulation of antigen-specific T cells

Stimulation of 1x10⁷ PBMCs with different antigens and isolation of CD154⁺ cells was performed as described above. Isolated CD154⁺ cells were cultured with mitomycin C (Sigma Aldrich) treated autologous feeder cells in a ratio of 1:100 at a density of 2.5x10⁶ cells/cm² in 48-well plates in X-Vivo15 (Lonza), supplemented with 5% (v/v) AB-serum (Lonza), 100 U/ml penicillin, 100 µg/ml streptomycin (Invitrogen) and 200 IU/ml IL-2 (Proleukin; Novartis). Cells were expanded for 14 days at 37°C in a humidified 5% (v/v) CO₂-containing atmosphere, culture medium was replenished and cells were split, when needed. After 7 and 14 days of expansion, cell numbers of the T cell lines were determined by flow cytometry. For re-stimulation, autologous PBMCs were CD3-depleted with a purity >99% using anti-CD3 microbeads and LD MACS columns (Miltenyi Biotec). CD3-depleted PBMCs were cryo-preservated in 70% X-Vivo15, 20% AB-serum, 10% DMSO (Sigma Aldrich) until re-stimulation of expanded T cell lines at day 14.

Two days before re-stimulation expanded T cell lines were harvested and adjusted in RPMI-1640 plus 5% (v/v) AB-serum without IL-2 to a final concentration of 1x10⁷/ml. For re-stimulation, 5x10⁵ expanded T cells were combined with 5x10⁵ thawed autologous CD3-depleted PBMCs and stimulated with different antigens, in presence of 1µg/ml CD28 functional grade pure Ab for 2+4h (Bref-A). After fixation and permeabilization cells were stained intracellular for CD4, CD154 and cytokine expression.

### Example 3: Aspergillus- and Candida-reactive CD4⁺ T cells belong to different T cell subsets

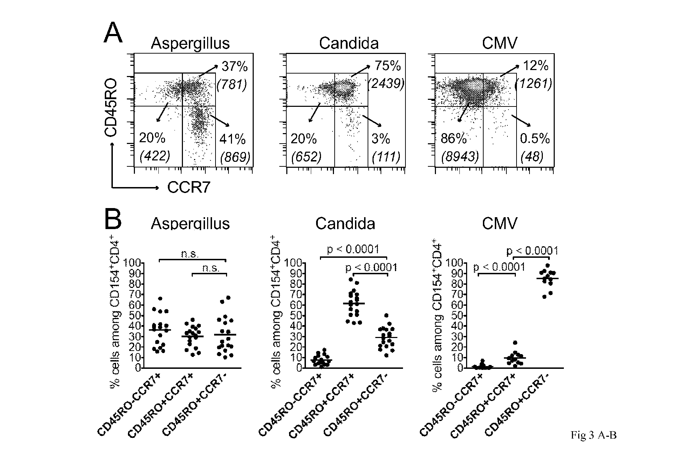

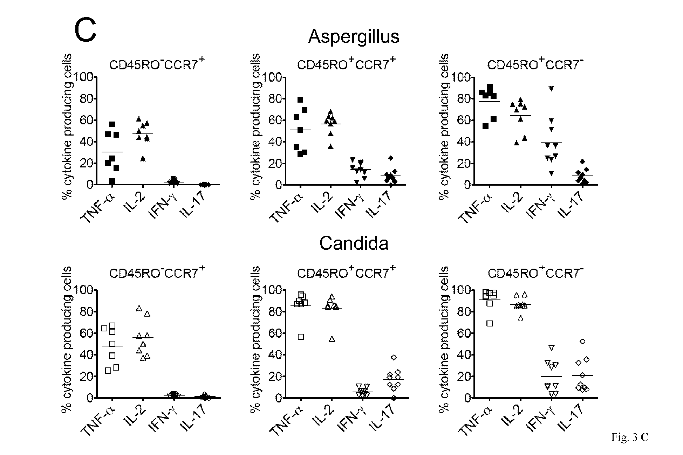

The quality of an immune response is not only affected by the frequency or absolute number of the antigen-specific cells but also by their function. In most situations the specific T cell pool is not uniform but can be split up in various functional subsets which further potentiates the need for an sensitive assay. Therefore we used the CD 154 enrichment to further characterize the fungus-reactive T cells directly *ex vivo.* As expected *Candida* and CMV stimulated T cells enriched for CD154⁺ mainly belonged to the CD45RO⁺ memory-type T cells (*Candida* 84.85 ± 15.99% and CMV 91.26 ± 7.30%) (Figure 3A, B). Further characterization showed, that the majority of *Candida*-reactive T cells belonged to the CD45RO⁺CCR7⁺ central memory subset (61.53 ±12.36%), whereas the CMV-reactive T cells displayed mainly a CD45RO⁻CCR7⁻ effector memory phenotype (85.24 ± 8.67%) as described before. Surprisingly, *Aspergillus-*reactive T cells also contained significant numbers of CD45RO⁻ cells (35 ± 16.46%). These CD45RO⁻ cells were *bona fide* naïve T cells, since they expressed CD45RA, CD27 (data not shown) and high levels of CCR7 but they did not secrete effector cytokines, like IFN-γ or IL-17 (Figure 3C). Regarding the memory subsets, *Aspergillus-*reactive T cells differed again from *Candida*-reactive T cells since they were distributed equally on both memory subsets (CD45RO⁺CCR7⁺ 31.8 ± 17.16%; CD45RO⁺CCR7⁻ 30.19 ± 9.78%) (Figure 3A, B).

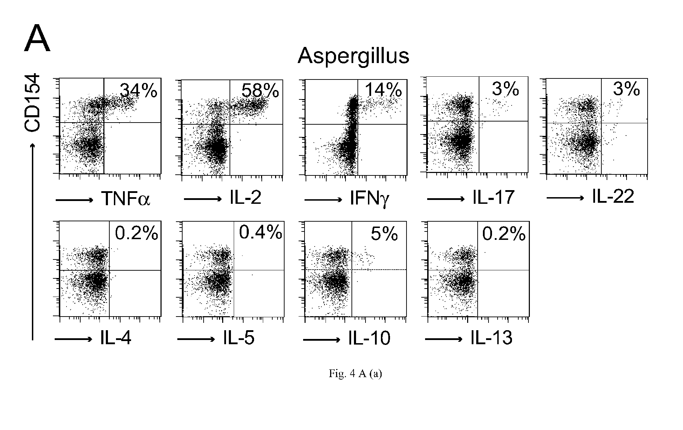

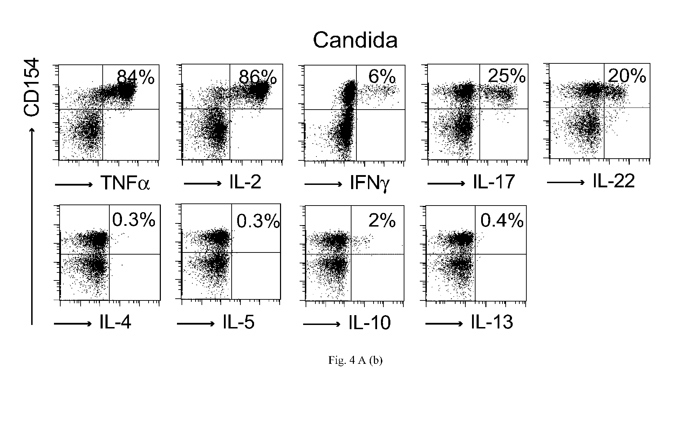

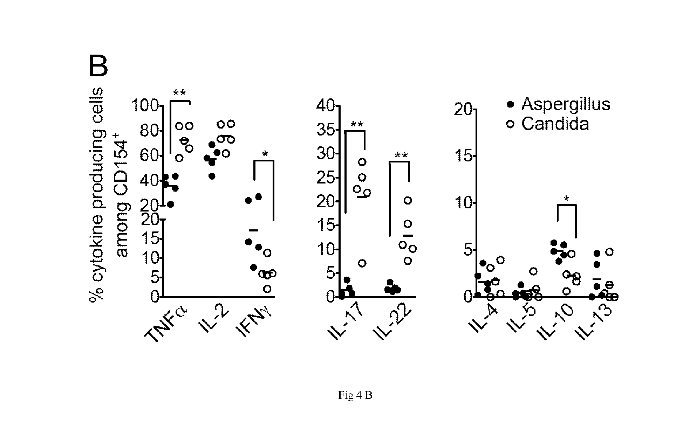

Next, we analyzed the cytokine profiles of *Aspergillus-* versus *Candida*-reactive T cells by intracellular staining following magnetic pre-selection of CD154⁺ T cells. As depicted in figure 1C, the magnetic pre-selection allowed collecting a significant number of antigen-reactive T cells, which even enabled the identification of rare cytokine producing subsets, such as IL-10. These cells would not be accessible without pre-enrichment. As summarized in figure 4A, B both *Aspergillus-* as well as *Candida*-stimulated CD4⁺ T cells produce high amounts of TNF-α (32.57 ± 11.30% and 71.28 ± 10.7%) and IL-2 (54.52 ± 11.15% and 73.57 ± 10.34%), whereas the frequency of the lineage-defining cytokines (IFN-γ, IL-17, IL-22, IL-4) was rather low (< 25%). Importantly, although T_{H}17 responses are often regarded as prototypic for fungal pathogens, we found clear differences between *Aspergillus-* and *Candida*-reactive CD4⁺ T cells. Whereas the response against *Aspergillus* was more of a T_{H}1 phenotype, with production of IFN-γ (15.03 ± 9.01%) and low IL-17 (1.40 ± 1.21%), IL-22 (1.65 ± 1.11%), *Candida*-reactive T cells contained a high amount of IL-17 (21.01 ± 7.3%) and IL-22 (12.27 ± 4.67%) producers, but only few IFN-γ expressing cells (5.81 ± 3.14%). Furthermore, a significant proportion of IL-10 producers (5.30 ± 1.63%) were detected in nearly all donors in response to *Aspergillus,* whereas the T_{H}2 cytokines IL-4, IL-5 and IL-13 were similarly low in response to both pathogens (0-4%).

Taken together, these results identify substantial differences in the surface phenotype as well as the cytokine production potential *of Aspergillus-* versus *Candida-*specific CD4⁺ T cells.

### Detailed description of figure 3

Reactive CD154⁺ T cells were *ex vivo* analysed for surface expression of CD45RO and CCR7 following magnetic enrichment upon activation with *Aspergillus, Candida* or CMV. (A) Dot plot examples for surface staining from one representative donor with percentages of cells among CD4⁺CD154⁺ lymphocytes and cell count (in brackets). (B) Summary of several donors *(Aspergillus, Candida* n=18, *CMV* n=12) with statistical significance of mean values, as determined by paired Student's t-test. Data from five independent experiments are shown. (C) Percentage of cytokine-expressing cells among the indicated phenotypic subsets gated on CD4+CD154+ T cells are shown for several donors, with horizontal lines indicating mean values. Three independent experiments were performed.

### Detailed description of figure 4

Flow cytometric analysis of cytokine expression after *Aspergillus-* (●) or *Candida-* (o) stimulation. Cells were stimulated and isolated as described in figure 3, followed by intracellular cytokine staining and analyzed for percentage of cytokine-expressing cells among CD154⁺ T cells. (A) Representative dot plot examples from one donor and (B) statistical analysis from several donors (n=5). Two independent experiments were performed; p values were determined using paired Student's t-test. * p < 0.5 ** p < 0.01

### Intracellular cytokine staining and characterization of phenotype by flow cytometry

PBMCs were stimulated and antigen-specific CD4⁺ T cells were isolated as described above. For phenotypic analysis enriched cells were stained with following mAbs (clone names in parantheses) according to manufacturer's protocols: antiBiotin-PE, antiBiotin-VioBlue (Bio3-18E7), CD4-Vioblue, CD4-FITC (VIT4), CD27-FITC, CD27-APC (M-T271), CD45RA-FITC (T6D11), CD45RO-APC (UCHL1) (Miltenyi Biotec), CCR7-PE (150503; R&D Systems), CD45RA-PerCP.Cy5.5 (HI100; Biolegend), CD45RO-FITC (UCHL-1; BD Pharmingen), CD45RO-PE.Cy7 (UCHL-1), CD4-PE.Cy7 (SK3) (BD Bioscience). After fixation and permeablization (Inside stain Kit; Miltenyi Biotec), CD154-expression was stained intracellular with CD154-VioBlue, CD154-APC, CD154-PE (5C8; Miltenyi Biotec), CD154-Allophycocyanin-eFluor780 (24-31; eBioscience), CD154-FITC (TRAP1; BD Pharmigen) alone, or in combination with different cytokines: TNFα-FITC (cA2), IFNγ-APC, IFNγ-FITC, IFNγ-PE (45-15), IL2-PE (N7.48A), IL4-PE (7A3-3), IL5-APC (JES1-39D10), IL10-APC (B-T10), IL13-PE (JES10-5A2.2), IL17-FITC (CZ8-23G1) (Miltenyi Biotec), IL22-PE (142928; R&D systems), IL22-PerCP.Cy5.5 (22URTI; eBiosecience), IFNγ-PerCP.Cy5.5 (4S.B3; BioLegend). Data were acquired on a MACSQuant analyzer and MACSQuantify software (Miltenyi Biotec) was used for analysis.

### Example 4: Increased frequencies of Aspergillus-reactive T cells in patients with invasive Aspergillosis

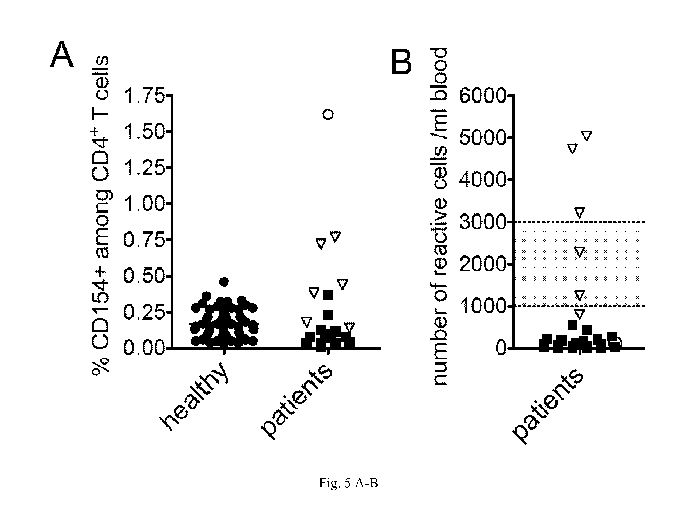

*Aspergillus* infections can be life threatening in immuno-compromised patients but so far only few diagnostic or prognostic immune-parameters exist. We used our assay in hematologic patients, which are at high risk for invasive Aspergillosis, to analyse whether changes in frequency or phenotype and function can be identified. In this patient group the frequency of CD4⁺ T cells can be massively altered, making the analysis of reactive cells by population-based assays, e.g. ELISA or ELISPOT, very imprecise. Therefore we used CD154-enrichment to directly isolate and quantitate the few fungus-reactive T cells. The frequency (Figure 5A) as well as cytokine pattern (data not shown) of *Aspergillus*-reactive T cells within the CD4⁺ T cell compartment is in the same range as in the healthy control group. However, due to the low frequency of CD4⁺ T cells in patient's peripheral blood the absolute numbers of *Aspergillus-reactive* T cells are almost 1-2 log reduced (Figure 5B). Interestingly, samples from two patients showed markedly increased frequencies of reactive T cells after *Aspergillus* stimulation and in fact these two patients were diagnosed for invasive Aspergillosis (Figure 5A).

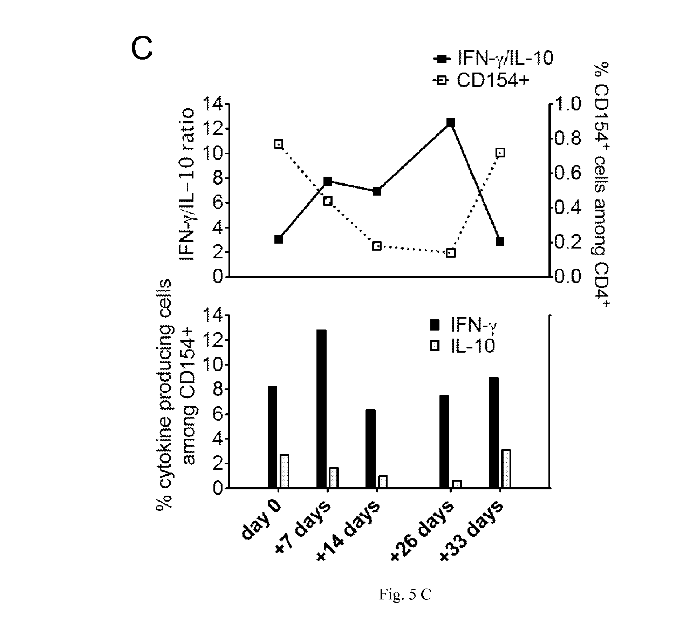

From one patient with diagnosed IA we had the opportunity to analyse several samples over a time period of 5 weeks. Throughout the observation period, the frequency of reactive T cells was variable but at 3 of 6 time points clearly higher than normal control values (0.5-0.8%) (Figure 5A, C). Despite the low absolute number of reactive T cells we could also determine the frequency of IFN-γ and IL-10 producers, since the IFN-γ/IL-10 ratio has previously been described as a prognostic parameter. Interestingly, especially IL-10 production was variable over time but directly correlated with the frequencies of *Aspergillus-*reactive T cells (Figure 5C). This suggests that the IFN-γ/IL-10 ratio as well as the total frequency of reactive cells may reflect an oscillating T cell response against *Aspergillus* in infected patients.

### Detailed description of figure 5

(A) Frequencies of *Aspergillus*-reactive T cells in peripheral blood of healthy donors (n=55) *versus* hematologic patients (n=17). Patients frequencies were calculated with the number of enriched CD154⁺ T cells and the number of stimulated CD4⁺ input cells Two patients with proven invasive Aspergillosis are depicted as white triangle and white circle, respectively. (B) Number of *Aspergillus*-reactive T cells per ml peripheral blood in hematologic patients. The range of healthy donors is marked in grey. (C) Kinetics of the *Aspergillus-*specific T cell response in one patient with proven invasive Aspergillosis (white triangle in figure A, B). The frequency of reactive cells (dotted line; right axis) as well as the IFN-γ/IL-10 ratio (dark line; left axis) and percentage of IFN-γ (dark grey) and IL-10 (light grey) producers among CD154+ (lower graph) were determined over a time period of 33 days.

### Example 5: Crohn's Disease patients show elevated responsiveness and IL-22 production against Candida

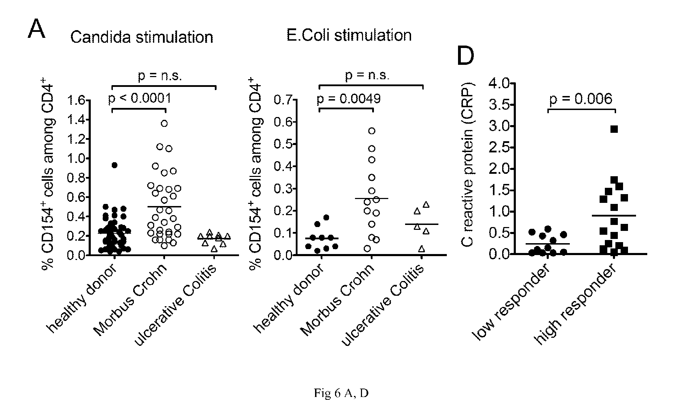

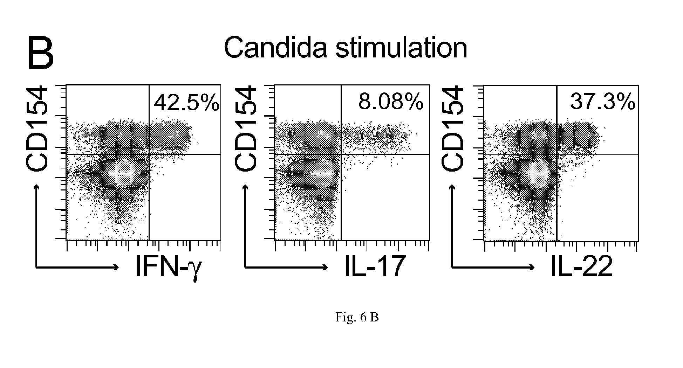

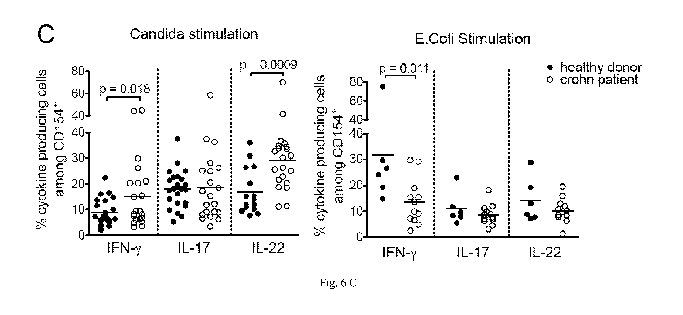

In patients with inflammatory bowel diseases an increased T cell reactivity against gut microbiota has been described. Since *Candida* is a common member of the gut microbiota, we hypothesized, that the *Candida*-reactive CD4⁺ T cell repertoire could be altered in patients with inflammatory bowel disease. Interestingly, patients with Crohn's disease (CD) showed a significant increase in the frequencies of *Candida-* as well as *E.coli*-reactive cells compared to healthy controls with a mean of 0.52% (p < 0.0001) and 0.26% (p = 0.0049) (Figure 6A). Further analysis of the cytokine production of *Candida*-reactive T cells revealed that IL-22 production was significantly increased in reactive cells from CD patients (Figure 6 B, C). Furthermore, some patients showed a markedly higher amount of IFN-γ producers and reduction of IL-17 producers, as compared to healthy donors, although these differences did not reach the level of significance. In addition, we found a significant decrease of IFN-γ producers in response to *E. Coli.* We further analysed whether reactivity to *Candida* correlates with clinical disease parameters. Indeed we found that patients with increased C-reactive protein serum levels are exclusively contained within the group of *Candida* high-responders (> 0.4% CD154⁺; Figure 6D).

### Detailed description of figure 6

(A) Frequencies of *Candida*-reactive T cells in peripheral blood of healthy donors (n=55) *versus* patients with Crohn's disease (n=31) or ulcerative colitis (n=9) as well as *E. Coli-*reactive T cells (healthy n=9, CD n=13, CU n=5). Significance was determined using unpaired Student's t-test. (B) Cytokine production of *Candida*-reactive T cells from one representative CD patient. Percentage of cytokine producing cells among CD154⁺ cells is indicated. (C) Summary of cytokine production from several patients compared to healthy controls with statistical significance of mean values, as determined by unpaired Student's t-test. (D) C-reactive protein serum levels of *Candida* high-responders (> 0.4% CD154⁺; n=16) versus low-responders (< 0.4% CD154; n=11). Significance was determined using unpaired Student's *t*-test.

### Example 6: In some patients with cystic fibrosis, the Aspergillus-reactive T cell response is biased towards a T_{H}2 cytokine pattern

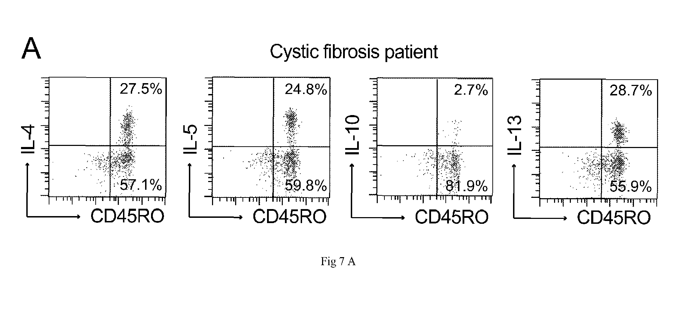

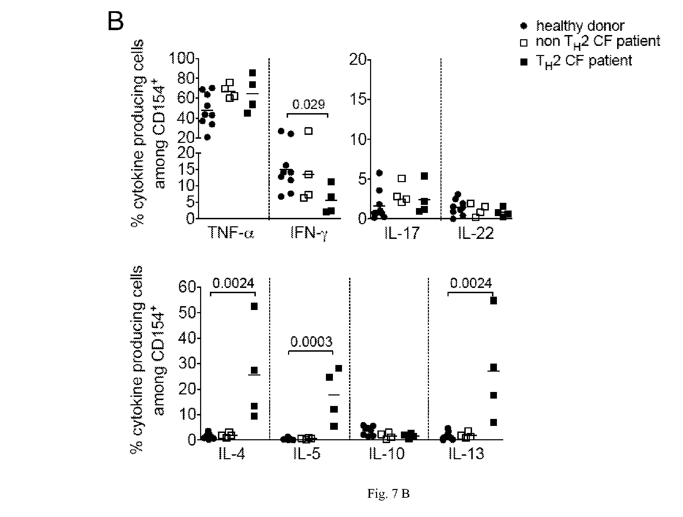

Patients with cystic fibrosis (CF) are characterized by an impaired mucociliary clearance, which can result in chronic colonisation with opportunistic pathogens, leading to allergic sensitation. For *Aspergillus* an increased IL-4 and IL-13 production has been described in patients with CF. We therefore analyzed the cytokine pattern of *Aspergillus-reactive* T cells from CF patients by our method. Indeed we found a strong expression of the T_{H}2 cytokines IL-4, IL-5 and IL-13 for some patients, comprising for up to 50% of the total *Aspergillus-*reactive T cell response, whereas other patients showed no alterations compared to healthy controls (Figure 7 A, B). In addition, the expression of IFN-γ was significantly reduced in patients with a T_{H}2 profile, whereas the expression of IL-10 as well as other cytokines did not differ (Figure 7B).

Taken together the analysis of three patient groups affected by fungal infections demonstrates that the sensitive analysis of fungus-reactive T cells based on CD154-enrichment is suitable to identify even subtle disease-associated alterations in the quantity and/or quality of antigen-specific T cells. Furthermore, our data highlight the possibility that monitoring fungus-specific T cell responses in patients may indeed be of diagnostic or prognostic value.

### Detailed description of figure 7

(A) Representative example of the *Aspergillus-*specific cytokine expression from one CF patient. Percentage of cytokine producing cells among CD154⁺ cells are indicated. (B) Summary of cytokine profiles from CF patients with T_{H}2 profile (n=4), without T_{H}2 profile (n=4) and healthy controls (n=9). Significance was determined using unpaired Student's t-test.

### Example 7: Enrichment and characterization of antigen-specific regulatory T cells via CD137

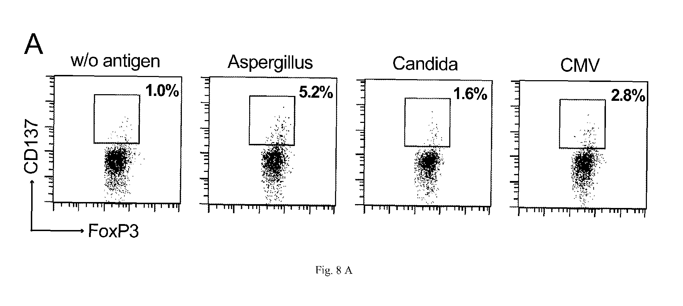

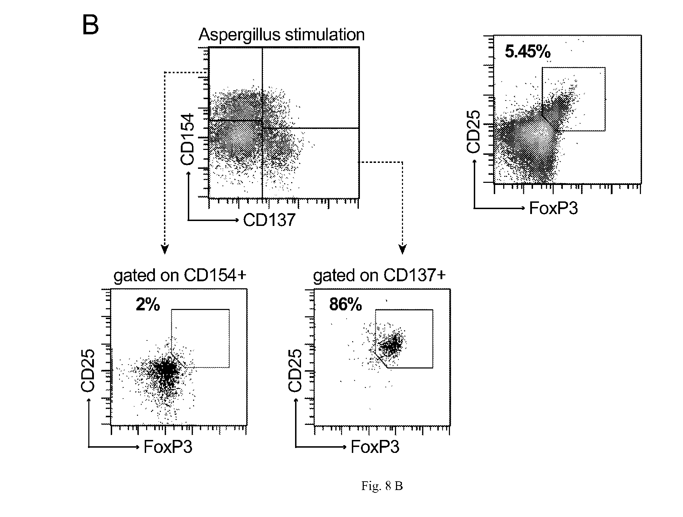

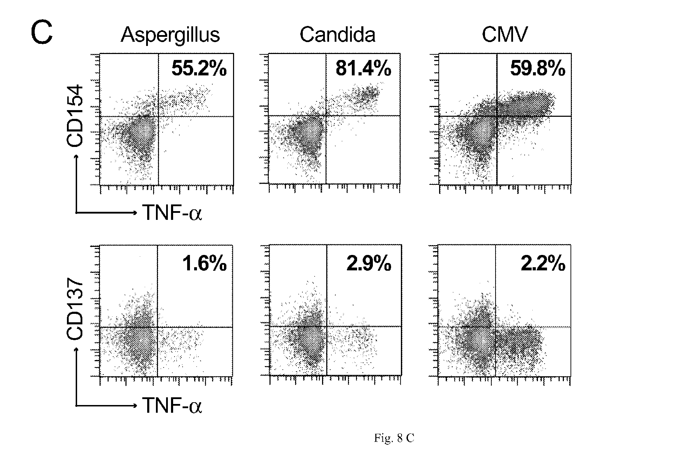

The combination of CD154 and CD137 can be used for the selection of antigen-specific Treg cells (EP appl. no. 10175578). Therefore we used the present invention to enrich and characterize CD137 expressing antigen-specific T cells. Stimulation with *Aspergillus, Candida* and *CMV* resulted in an increased expression of CD137 on CD25+FoxP3+ regulatory T cells (Figure 8A). To further characterize these cells, antigen specific T cells were isolated according to CD154 and CD137 expression and stained for the regulatory T cell markers CD25 and FoxP3. As shown in figure 8B, CD137 enrichment resulted in a sizable population of Treg cells, whereas within the CD154⁺ population CD25⁺FoxP3⁺ cells could only marginally be detected. Next, we analyzed the cytokine expression of CD154- versus CD137-expressing cells by intracellular staining after combined magnetic pre-selection. As shown in figure 8C, only CD154-expressing T cells produced TNF-α after *Aspergillus-, Candida-* and *CMV*-stimulation, whereas CD137⁺ cells showed nearly no TNF-α production. These results demonstrate, that our method can be used for the detection and characterization of antigen-specific Tregs, as well as to define major differences compared to conventional T cells.

### Detailed description of figure 8

(A) PBMCs were stimulated with the indicated antigens and analysed for CD137-expression among CD4⁺CD25⁺FoxP3⁺ T cells. Dot plot example for one representative donor out of 40 is shown. (B) Magnetically enrichment of CD154⁺ and CD137⁺ T cells followed by CD25 and FoxP3 staining. Percentage of CD25⁺FoxP3⁺ T cells among enriched CD154⁺ or CD137⁺ are indicated. (C) Representative example for intracellular cytokine staining of CD154⁺ or CD137⁺ cells after combined enrichment. Numbers indicated percentage of cytokine positive cells among activation marker positive CD4⁺ cells.

### Combined isolation of antigen-specific CD137⁺ and CD154⁺ T cells and FoxP3 staining

For separation of antigen-specific T cells, 1x10⁷ PBMCs/ml were stimulated with the different antigens at 37°C in a 5% (v/v) CO₂ atmosphere. To prevent down-regulation of CD154 expression, 1µg/ml CD40 functional grade pure antibody (Miltenyi Biotec) was added. If separation of antigen-specific T cell was combined with an intracellular staining, 1µg/ml Brefeldin A (Sigma Aldrich) was added for the last 2 hours of stimulation.

After stimulation, cells were separated using the CD154 MicroBead Kit and the CD137 MicroBead Kit (Miltenyi Biotec). In brief, cells were indirectly magnetically labelled with CD154-Biotin and anti-Biotin microbeads as well as CD137-PE and anti-PE microbeads in each case with a titer of 1:10 and enriched by two MS MACS columns (Miltenyi Biotec). For characterization of phenotypic markers, surface staining was performed onto the second column. For FoxP3 and cytokine staining, surface marker-stained cells were fixed, permebealized, and stained for intranuclear FoxP3 and cytokines using FoxP3 staining kit (MiltenyiBiotec) according to manufacturer's instructions.

### Example 9: Enrichment of tumor/autoantigens-antigen-reactive T cells from peripheral blood of healthy donors.

Certain autoantigens have been described to be strongly expressed in certain tumor cells, e.g. Melan A, WT-1 and NY-ESO. Characterisation of the response towards these antigens in healthy persond or tumor patients might be important to predict the outcome of the antitumor response or may be used to identify certain antigens/epitopes, e.g, which have already high precursor frequency, which may predict the success of anti tumor vaccination with a certain antigen.

### Detailled description of Fig. 9

10⁷ cells from freshly isolated PBMC, cultivated in 1 mL RPMI-1640 with glutamine and 5% AB serum overnight, and were stimulated using either a mixture of three tumor-antigen peptide pools (PepTivators for: WT-1, MelanA and NY-ESO-1) or without the addition of a stimulus as negative control. For the incubation anti-CD28 and anti-CD40 antibodies are added at a concentration of 1µg/mL. Anti-CD28 is used to ensure delivery of co-stimulatory signals whereas anti-CD40 should ensure that CD 154 (CD40L) is not down-regulated due to cellular interactions with CD40 on APCs. After stimulation a 10% aliquot is taken, stained and analyzed by flow cytometry (left and center panels). The remainder of the cells is labelled with anti-CD154-biotin and subsequently with anti-biotin-Microbeads, followed by magnetic cell separation and characterization (right panels). The dotplots depicted for the original fraction are gated on CD4+ lymphocytes, the dotplots for the enriched fraction on viable CD4+ lymphocytes. As can be seen in figure 9 without enrichment no significant difference in the frequency of cells expressing CD154 and/or CD69 can be observed with and without antigen stimulation. However, after enrichment about 100 CD154+/CD69+ T cells can be detected in the antigen stimulated sample whereas no cells are isolated without antigen. The co-expression of CD69 further indicates that the cells are really activated T cells. About 100 cells were isolated from 10⁷ starting cells, indicating a frequency of 1 in 10⁵ in a healthy subject.

### Detailled description of Fig. 10

To obtain larger cell numbers for more detailed analysis the same experiment was repeated using a 10fold higher starting cell number. 10⁸ cells from freshly isolated PBMC of CMV-positive donors, cultivated in RPMI-1640 with glutamine and 5% AB serum overnight, and were stimulated using either a mixture of three tumor-antigen peptide pools (PepTivators for: WT-1, MelanA and NY-ESO-1) or without the addition of a stimulus as negative control. As a positive control 5x10⁷ cells are stimulated in the same manner with a mixture of two CMV peptide pools (PepTivators for pp65 and IE-1). For the incubation anti-CD28 and anti-CD40 antibodies were added at a concentration of 1µg/mL. Anti-CD28 was used to ensure delivery of co-stimulatory signals whereas anti-CD40 was used to ensure that CD154 (CD40L) is not downregulated due to cellular interactions with CD40 on APCs. After stimulation the cells are harvested and labeled with anti-CD154-biotin and subsequently with anti-biotin-Microbeads, followed by magnetic cell separation. The positive fraction of the separated cells are stained and analyzed by flow cytometry for further activation induced cytokines IFN- y and, TNF-α, to identify these functional subsets. The dotplots depicted are gated on CD4+ lymphocytes. As can be seen in figure 10 after enrichment about 1000 CD154+/cytokine+ T cells can be detected in the antigen stimulated sample whereas only about 100 cells are isolated without antigen. The co-expression of cytokines indicates that the cells are really activated T cells and also shows that the total population can be further subdivided into certain small functional subsets. About 1000 cells were isolated from 10⁸ starting cells, indicating a frequency of 1 in 10⁵ in a healthy subject.

## Claims

1. Method for sensitive quantitative and/or qualitative analysis of target T cells comprising the steps
a) enrichment of said cells from a mixture of said cells and other cells in a sample by the use of one or more activation markers expressed on antigen-activated T cells in a parallel cell sorting process; and
b) analysis of the cells of step a).

2. Method of claim 1 wherein the parallel cell sorting process is a magnetic cell sorting process.

3. Method of claim 1 or 2 wherein the magnetic cell sorting process uses superparamagnetic beads and separation columns.

4. Method of claim 3 wherein manipulations of the target T cells following step a) are directly performed on the separation column.

5. Method of any one of claims 1 to 4 wherein the activation markers are CD137 and/or CD154 and/or cytokines.

6. Method of any one of claims 1 to 5 wherein the analysis of step b) is flow cytometry.

7. Method of any one of claims 1 to 6 wherein the target T cells are specific for pathogens, tumor antigens, environmental antigens or autoantigens.

8. Method of any one of claims 1 to 7 wherein the target T cells are specific for fungi.

9. Method of any one of claims 1 to 8 wherein the target T cells are specific for Aspergillus fumigatus and Candida albicans.

10. Method according to anyone of claim 1 to 9 for diagnosing fungal infections in animals.

11. Method according to anyone of claim 1 to 7 for diagnosing autoimmune diseases in animals

12. Method according to anyone of claim 1 to 7 for diagnosing allergies in animals

13. Method according to anyone of claim 1 to 7 for diagnosing tumor diseases in animals

14. Method according to anyone of claim 1 to 8 for monitoring or predict the success of vaccination with a certain antigen.
